(19) Europäisches Patentamt
European Patent Office
Office européen des brevets

(11) **EP 3 812 041 A1**

(12) **EUROPEAN PATENT APPLICATION**

(43) Date of publication:
**28.04.2021 Bulletin 2021/17**

(51) Int Cl.:
**B01L 3/00** *(2006.01)*   **C12Q 1/6869** *(2018.01)*
**G01N 33/543** *(2006.01)*

(21) Application number: **19205485.6**

(22) Date of filing: **25.10.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sharp Life Science (EU) Limited Oxford, Oxfordshire OX4 4GB (GB)**

(72) Inventors:
• **BROWN, Christopher James Oxford, Oxfordshire OX4 4GB (GB)**
• **HADWEN, Benjamin James Oxford, Oxfordshire OX4 4GB (GB)**

(74) Representative: **Suckling, Andrew Michael Marks & Clerk LLP Fletcher House The Oxford Science Park Heatley Road Oxford OX4 4GE (GB)**

(54) **METHOD OF OPERATING EWOD DEVICE WITH SENSING APPARATUS**

(57)    A method of determining a characteristic of a first pair of droplets in an electrowetting on dielectric microfluidic device substantially free from the influences of interference, comprises forming a droplet interface bilayer between a first pair of droplets, a droplet of the first pair of droplets contacting a first sensing electrode and another droplet of the first pair of droplets contacting a first bias electrode. A droplet interface bilayer is formed between a second pair of droplets, a droplet of the second pair of droplets contacting a second sensing electrode and another droplet of the second pair of droplets contacting a second bias electrode. A nanopore is inserted into the droplet interface bilayer of the first pair of droplets, a first signal is measured between the first bias electrode and the first sensing electrode due to transport of a target species through the nanopore of the first pair of droplets and a first signal is measured between the second bias electrode and the second sensing electrode for the second pair of droplets. A difference between the first measured signal from the first pair of droplets and the first measured signal from the second pair of droplets is measured, and the difference between the first measured signal from the first pair of droplets and the first measured signal from the second pair of droplets is reported as being the characteristic of the first pair of droplets, substantially free from interference.

Figure 10

## Description

## Technical Field

The present invention relates to a microfluidic device, and to methods of determining a characteristic of a fluid package in such a device. More particularly, the invention relates to methods of operating an Electro-wetting on Dielectric (EWOD) microfluidic device such as, for example, an Active Matrix Electro-wetting on Dielectric (AM-EWOD) microfluidic device. Electro-wetting-On-Dielectric (EWOD) is a known technique for manipulating droplets of fluid on an array. Active Matrix EWOD (AM-EWOD) refers to implementation of EWOD in an active matrix array incorporating transistors, for example by using thin film transistors (TFTs).

## Background Art

Microfluidics is a rapidly expanding field concerned with the manipulation and precise control of fluids on a small scale, often dealing with sub-microlitre volumes. There is growing interest in its application to chemical or biochemical assays and synthesis, both in research and production, and applied to healthcare diagnostics ("lab-on-a-chip"). In the latter case, the small nature of such devices allows rapid testing at point of need using much smaller clinical sample volumes than for traditional lab-based testing.

A microfluidic device has one or more channels (or more generally gaps) with at least one dimension less than 1 millimeter (mm). Common fluids used in microfluidic devices include whole blood samples, bacterial cell suspensions, protein or antibody solutions and various buffers. Microfluidic devices can be used to obtain a variety of interesting measurements including molecular diffusion coefficients, fluid viscosity, pH, chemical binding coefficients and enzyme reaction kinetics. Other applications for microfluidic devices include capillary electrophoresis, isoelectric focusing, immunoassays, enzymatic assays, flow cytometry, sample injection of proteins for analysis via mass spectrometry, PCR amplification, DNA analysis, cell manipulation, cell separation, cell patterning and chemical gradient formation. Many of these applications have utility for clinical diagnostics.

Many techniques are known for the manipulation of fluids on the sub-millimeter scale, characterised principally by laminar flow and dominance of surface forces over bulk forces. Most fall into the category of continuous flow systems, often employing cumbersome external pipework and pumps. Systems employing discrete droplets instead have the advantage of greater flexibility of function.

Electro-wetting on dielectric (EWOD) is a well-known technique for manipulating discrete droplets of fluid by application of an electric field. It is thus a candidate technology for microfluidics for lab-on-a-chip technology. An introduction to the basic principles of the technology can be found in "Digital microfluidics: is a true lab-on-a-chip possible?" (R. B. Fair, Microfluid Nanofluid (2007) 3:245-281).

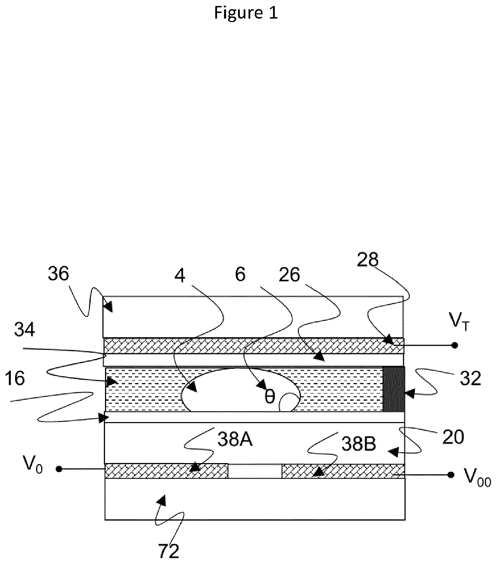

Fig. 1 shows a part of a conventional EWOD device in cross section. The device includes a lower substrate 72, on which is provided a conductive material which is patterned so that a plurality of array element electrodes 38 (e.g., 38A and 38B in Fig. 1) are realized. The electrode of a given array element may be termed the element electrode 38. The liquid droplet 4, including a polar material (which is commonly also aqueous and/or ionic), is constrained in a plane between the lower substrate 72 and a top substrate 36. A suitable gap between the two substrates may realize a fluid chamber between the two substrates, created, for example, by means of a spacer 32. A non-polar fluid 34 (e.g. oil) may be used to occupy that part of the volume of the fluid chamber not occupied by the liquid droplet 4. An insulator layer 20 disposed upon the lower substrate 72 separates the conductive element electrodes 38A, 38B from a first hydrophobic coating 16 upon which the liquid droplet 4 sits with a contact angle 6 represented by $\theta$. The hydrophobic coating is formed from a hydrophobic material (commonly, but not necessarily, a fluoropolymer).

On the top substrate 36 is a second hydrophobic coating 26 with which the liquid droplet 4 may come into contact. Interposed between the top substrate 36 and the second hydrophobic coating 26 is a reference electrode 28.

The droplets have a contact angle 6 with the hydrophobic surface of the insulator layer. The contact angle 6 is determined by the balancing of the surface tension components (1) from the hydrophobic coating 16 to the liquid of the droplets 4 ($\gamma SL$) interface, (2) from the liquid of the droplets 4 to the surrounding fluid medium 34 ($\gamma LG$) interface, and (3) from the hydrophobic coating 16 to the surrounding fluid medium 34 ($\gamma SG$) interface. Where no voltages are applied, the contact angle 6 satisfies Young's law, and is of size $\theta$ given by the equation:

$$\cos \theta = ((\gamma SG - \gamma SL) / \gamma LG) \text{ (Equation 1).}$$

In operation, voltages termed the EW drive voltages, (e.g. $V_T$, $V_0$ and $V_{00}$ in Fig. 1) may be externally applied to different electrodes (e.g. reference electrode 28, element electrodes 38, 38A and 38B, respectively). The resulting electrical forces that are set up effectively control the hydrophobicity of the hydrophobic coating 16. By arranging for different EW drive voltages (e.g. $V_0$ and $V_{00}$) to be applied to different element electrodes (e.g. 38A and 38B), the liquid droplet 4 may be moved in the lateral plane between the two substrates 72 and 36.

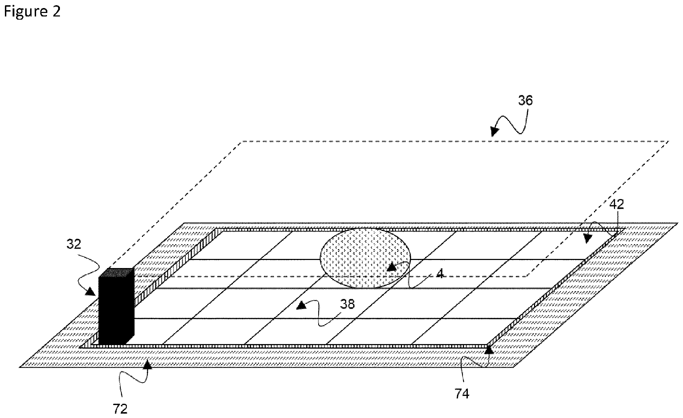

Fig. 2 is a drawing depicting additional details of an exemplary AM-EWOD device in schematic perspective, which may incorporate the layered structures in Fig. 1. The AM-EWOD device has a lower substrate 72 with thin film electronics 74 disposed upon the lower substrate 72, and a reference electrode (not shown, but comparable to reference electrode 28 above) is incorporated into an upper substrate 36 (corresponding to the upper substrate of Fig. 1). (Alternatively the electrode configuration may be reversed from that shown in Fig. 2, with the thin film electronics being incorporated into the upper substrate and the reference electrode being incorporated into the lower substrate.) The thin film electronics 74 are arranged to drive array element electrodes 38 - for example the thin film electronic 74 associated with an array element electrode may comprise one or more thin-film transistors (TFTs) that are controlled by an EWOD control unit (not shown). A plurality of array element electrodes 38 are arranged in an electrode or element array 42, having X by Y array elements where X and Y may be any integer. A liquid droplet 4 which may include any polar liquid and which typically may be aqueous, is enclosed between the lower substrate 72 and the upper substrate 36 separated by a spacer 32, although it will be appreciated that multiple liquid droplets 4 can be present.

As described above with respect to the representative EWOD structure, the EWOD channel or gap defined by the two substrates initially is filled with the nonpolar filler fluid (e.g. oil). The liquid droplets 4 including a polar material, i.e., the droplets to be manipulated by operation of the EWOD device, must be inputted from an external "reservoir" of fluid into the EWOD channel or gap. The external reservoir may for example be a pipette or may be a structure incorporated into the plastic housing of the device. As the fluid from the reservoir for the droplets is inputted, filler fluid gets displaced and is removed from the EWOD channel.

Example configurations and operation of EWOD devices are described in the following. US 6911132 (Pamula et al., issued June 28, 2005) discloses a two-dimensional EWOD array to control the position and movement of droplets in two dimensions. US 6565727 (Shenderov, issued May 20, 2003) further discloses methods for other droplet operations including the splitting and merging of droplets, and the mixing together of droplets of different materials. US 7163612 (Sterling et al., issued Jan. 16, 2007) describes how TFT based thin film electronics may be used to control the addressing of voltage pulses to an EWOD array by using circuit arrangements very similar to those employed in AM display technologies.

EWOD devices are useful for miniaturising and automating biochemical/chemical processes through the manipulation of aqueous droplets in an oil matrix; EWOD devices may be used to split, move, merge and mix droplets. Typically, biochemical/chemical workflows involve carrying out a sequence of reactions on biological/chemical samples, with each step involving the merging of one or more droplets containing the reagents necessary for that step with the sample droplet. The process of merging of droplets containing reagents into the sample droplet means that the volume of the sample droplet typically increases as the workflow proceeds, and the reaction droplet's composition becomes the sum of the workflow product(s) and any remaining reactants and by-products.

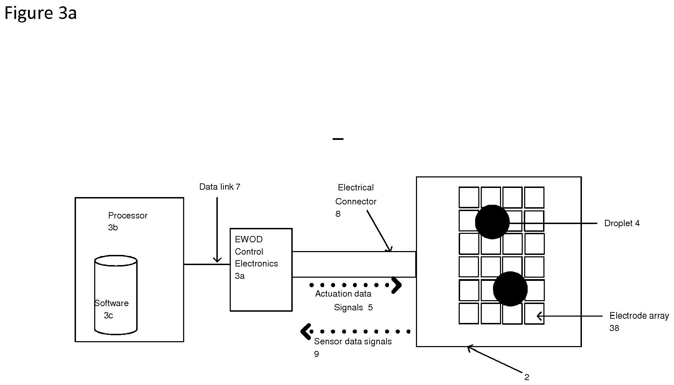

The EWOD device is typically operated as part of a microfluidic system comprising a control system configured to control actuation voltages applied to the electrode array of the EWOD device (or other microfluidic device) to perform manipulation operations to the fluid droplets. For example, Fig. 3a is a block schematic diagram of a microfluidic system 1. The system comprises an EWOD (or other microfluidic) device 2, in this example an active matrix EWOD (AM-EWOD) device, and a controller unit 3. The controller unit comprises electrode control electronics 3a and a processor 3b running appropriate software 3c. A storage device (not shown) may store any application software and any data associated with the system. The electrode control electronics generates, under control of the processor 3b, actuation voltages 5 for applying to element electrodes 38 and a common electrode (not shown) of the microfluidic device 2, for example to effect a pre-determined sequence of droplet operations. Fig. 3a shows the processor 3b as separate from the control electronics 3a and communicating with the control electronics 3a over a datalink 7, but the processor 3b and the control electronics 3a could alternatively be integral with one another. Fig. 3a shows the electrode control electronics 3a as separate from the EWOD device 2 and communicating with the EWOD device via an electrical connector 8, but the control electronics 3a and the EWOD device 2 could alternatively be integral with one another. The control electronics may include suitable circuitry and/or processing devices that are configured to carry out various control operations relating to control of the EWOD device, such as a CPU, microcontroller or microprocessor.

The control electronics 3a may further receive data signals 9 from one or more sensors (not shown) of the EWOD device 2. The sensor data signals 9 may include signals obtained by operating the EWOD array elements in a sensing mode, and/or may include signals obtained by one or more sensors external to the EWOD array elements such as some or all of illumination and/or detection optics, a thermal control unit or magnetic systems for interacting with the droplets 4.

There are several known methods of measuring the electrical properties of droplets in an EWOD device. Schetrzer (Sensors and Actuators B 145 (2010) 340-347) describes how (complex) impedance measurement through the bottom substrate electrodes can determine droplet conductivity. US20140194305 (Kayyem, Genmark) also describes an electrowetting device with detection electrodes on the bottom substrate to perform electrochemical sensing. US7163612 (Sterling, Keck Grad. Inst.) describes how sensor circuitry may be integrated onto the top substrate. WO2019126715 A1 discloses a method of measuring the electrical properties of an interface between two droplets.

## Summary

A first aspect of the present invention provides a method of determining a characteristic of a first pair of droplets in an electrowetting on dielectric microfluidic device substantially free from the influences of interference, the method comprising; forming a droplet interface bilayer between a first pair of droplets, a droplet of the first pair of droplets contacting a first sensing electrode and another droplet of the first pair of droplets contacting a first bias electrode; forming a droplet interface bilayer between a second pair of droplets, a droplet of the second pair of droplets contacting a second sensing electrode and another droplet of the second pair of droplets contacting a second bias electrode; inserting a transporter species into the droplet interface bilayer of the first pair of droplets; measuring a first signal between the first bias electrode and the first sensing electrode due to transport of a target species through the transporter species of the first pair of droplets and measuring a first signal between the second bias electrode and the second sensing electrode for the second pair of droplets; determining a difference between the first measured signal from the first pair of droplets and the first measured signal from the second pair of droplets; and reporting the difference between the first measured signal from the first pair of droplets and the first measured signal from the second pair of droplets as being the characteristic of the first pair of droplets, substantially free from interference. In this aspect a signal that contains a signal component of interest is measured for the first pair of droplets, and a signal that does not contain the signal component of interest but that is expected to contain similar noise or interference components is measured for the second pair of droplets, for use in correcting the measured signal for the effects of noise/interference. The signal component of interest arises from transport of a species through the transporter species.

It should be understood that referring to a droplet "contacting" an electrode refers to the droplet making electrical contact with the electrode, and does not require the droplet to make physical contact with the electrode. In particular, this wording does not exclude the possibility that a thin hydrophobic layer is provided over the electrode and that the droplet is capacitively coupled to the electrode via the hydrophobic coating.

The measured first signal of the first pair of droplets may comprise a signal component of interest and a first signal component of interference; and wherein the first signal of the second pair of droplets comprises a second component of interference.

The signal component of interest in the measured first signal of the first pair of droplets may be indicative of passage of a first species through the transporter species.

The method may comprise inserting a transporter species into the droplet interface bilayer of the second pair of droplets, wherein the first species is absent
from the second pair of droplets. Alternatively, no transporter species may be present in the droplet interface bilayer of the second pair of droplets and in this case, provided that the first species does not pass through the droplet interface bilayer, the first species may be present in one or both droplets of the second pair of droplets

The first pair of droplets and the second pair of droplets have a droplet in common.

The first bias electrode may be continuous with the second bias electrode, to form a common bias electrode. Alternatively, the first and second bias electrodes may be separate electrodes that are addressable independently of one another.

The method may comprise transferring the first measured signal from the first pair of droplets and the first measured signal from the second pair of droplets to a signal processor configured to determine the difference between the first measured signal from the first pair of droplets and the first measured signal from the second pair of droplets.

Forming the droplet interface bilayer between the first pair of droplets may comprise manipulating a first pair of droplets to form the first droplet interface bilayer (DIB) positioned such that the droplet of the first pair of droplets contacts the first sensing electrode and the another droplet of the first pair of droplets contacts the first bias electrode; and wherein forming the droplet interface bilayer between the second pair of droplets may comprise manipulating a second pair of droplets to form the second droplet interface bilayer (DIB) positioned such that the droplet of the second pair of droplets contacts the second sensing electrode and the another droplet of the second pair of droplets contacts the second bias electrode.

A second aspect of the present invention provides a method of determining a characteristic of a first pair of droplets in an electrowetting on dielectric microfluidic device substantially free from the influences of interference, comprising; forming a droplet interface bilayer between a first pair of droplets; contacting a droplet of the first pair of droplets with a first sensing electrode and contacting another droplet of the first pair of droplets with a first bias electrode; measuring a first signal between the first bias electrode and the first sensing electrode for the first pair of droplets; inserting a transporter species into the droplet interface bilayer of the first pair of droplets; measuring a second signal between the first bias electrode and the first sensing electrode due to transport of a target species through the transporter species of the first pair of droplets; determining a difference between the second measured signal from the first pair of droplets and the first measured signal from the first pair of droplets; and reporting the second measured signal from the first pair of droplets and the first measured signal from the first pair of droplets as being the characteristic of the first pair of droplets, substantially free from interference. A method of this aspect is generally complementary to a method of the first aspect, in that a signal that contains a signal component of interest is measured for one pair of droplets, and a signal that does not contain the signal component of interest but that is expected to contain similar noise or interference components is also obtained for use in correcting the measured signal for the effects of noise/interference. In a method this aspect however the two signals are obtained sequentially using a single droplet pair (by obtaining one signal when no transporter species is present in the droplet interface bilayer of the droplet pair and obtaining another signal when a transporter species is present in the droplet interface bilayer of the droplet pair), whereas in a method of the first aspect the two signals are obtained using different droplet pairs (and may be acquired simultaneously).

The first signal of the first pair of droplets may comprise a first component of interference and the measured second signal of the first pair of droplets may comprise a signal component of interest and a second signal component of interference. Thus, the difference between the first signal and the second signal represents the signal component of interest substantially free from interference.

The signal component of interest in the measured second signal of the first pair of droplets may be indicative of passage of a first species through the transporter species.

The method may further comprise applying a potential across the transporter species in the droplet interface bilayer of the first pair of droplets to thereby promote passage of the first species through the transporter species. This potential may for example be applied via the first bias electrode and the first sensing electrode.

In any aspect or embodiment the transporter species may be a nanopore.

The transporter species (nanopore) may be selective for the transfer of nucleic acid between one droplet and the other droplet of the first pair of droplets. If a nucleic acid is present at different concentrations in the droplets of the first pair of droplets the nucleic acid passes through the transporter species (nanopore), and this transfer of the nucleic acid from one droplet to another enables nucleotide sequencing of the nucleic acid. Conveniently, the nucleic acid is present in one droplet of the droplet pair (which may be considered as a "source" droplet), and is not present in the other droplet of the first droplet pair.

The transporter species (nanopore) may be selective for the transfer of protein between one droplet and the other droplet of the pair of droplets. If a protein is present at different concentrations in the droplets of the first pair of droplets (and conveniently is present in one droplet of the droplet pair (which may be considered as a "source" droplet), and is not present in the other droplet of the first droplet pair) the protein passes through the transporter species (nanopore), and this transfer of the protein from one droplet to another enables determination of the presence (or absence, in the event of no observed transfer) of a specific protein, and/or determination of the concentration of the protein in the source droplet.

The transporter species (nanopore) may be selective for the transfer of ionic species between one droplet and the other droplet of the first pair of droplets. If an ionic species acid is present at different concentrations in the droplets of the first pair of droplets (and conveniently is present in one droplet of the droplet pair (which may be considered as a "source" droplet), and is not present in the other droplet of the first droplet pair) the ionic species passes through the transporter species (nanopore), and this transfer enables determination of the ionic composition of the source droplet.

A further aspect provides a method of performing nucleic acid sequencing through a nanopore (or other transporter species) within an electrowetting on dielectric (EWOD) microfluidic device, comprising; providing a plurality of droplets within a chamber of the EWOD microfluidic device; manipulating a first pair of droplets to form a first droplet interface bilayer (DIB); manipulating a second pair of droplets to form a second DIB; positioning the first DIB and the second DIB such that the respective droplets make contact with a first pair of electrodes and a second pair of electrodes respectively; inserting a pore (or other transporter species) into the first DIB and optionally inserting a pore into the second DIB; applying a potential across the pore in the first DIB with the first pair of electrodes, and applying a potential across the pore in the second DIB with the second pair of electrodes; measuring a first current arising from transfer of a nucleic acid through the pore in the first DIB and measuring a second current arising due to influence of noise in the second DIB; processing the measured first and second currents to yield a processed current that is substantially free from noise; wherein the processed current is a more accurate representation of the nucleic acid sequence as compared with a measurement obtained with a second DIB is used to compensate the influence of noise.

Preferred embodiments of the present invention will now be described by way of illustrative examples with reference to the accompanying figures, in which:

Fig. 1 is a schematic cross-sectional view of an EWOD device;
Fig. 2 is a schematic perspective view of an EWOD device;
Fig. 3a is a schematic view of an EWOD system;
Fig. 3b is a schematic view of an EWOD system with improved sensing electronics;
Fig. 4a is a plan view of an exemplary top plate of an EWOD device with integrated sensing electrodes;
Fig. 4b is a plan view of another exemplary top plate of an EWOD device with integrated sensing electrodes.
Fig. 4c is a plan view of yet another exemplary top plate of an EWOD device with integrated sensing electrode, showing a connector.
Fig. 5 represents a first embodiment of a sensor configuration with a differential circuit.

Fig. 6a represents a cross sectional view through an exemplary EWOD device depicting relationship between a single droplet within chamber and respective electrodes with a patterned hydrophobic coating.

Fig. 6b represents a cross sectional view through an exemplary EWOD device depicting relationship between a single droplet within chamber and respective electrodes without a patterned hydrophobic coating.

Fig. 7a represents a first differential circuit configuration.

Fig. 7b represents a second differential circuit configuration.

Fig. 7c represents a third differential circuit configuration.

Fig. 7d represents a fourth differential circuit configuration.

Fig. 8 represents a second embodiment of a sensor configuration with a differential circuit.

Fig. 9 represents a third embodiment of a sensor configuration with a differential circuit.

Fig. 10 represents an fourth embodiment of a sensor configuration with a differential circuit.

Fig. 11 represents a fifth embodiment of a sensor configuration with a differential circuit.

Fig. 12 represents a sixth embodiment of a sensor configuration with a differential circuit.

Fig. 13a-b represent cross sectional views through an exemplary EWOD device depicting relationship between droplets within chamber and respective electrodes.

Detailed Description of embodiments

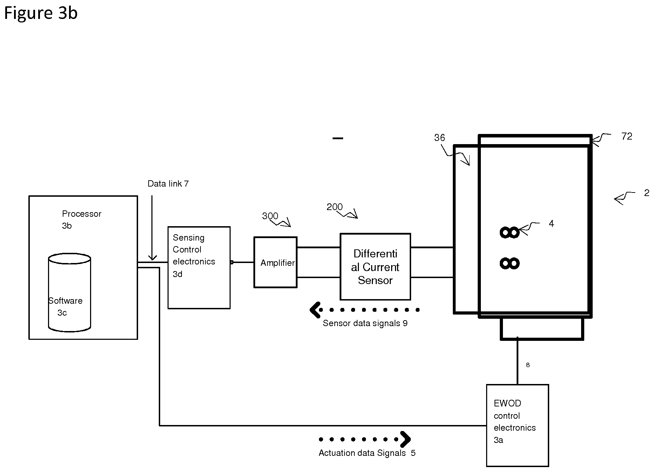

Fig. 3b is a a block schematic diagram of a microfluidic system 1 having an improved sensor circuit. The system comprises an EWOD (or other microfluidic) device 2, in this example an active matrix EWOD (AM-EWOD) device, and a controller unit 3. The controller unit comprises EWOD control electronics 3a and a processor 3b running appropriate software 3c. A storage device (not shown) may store any application software and any data associated with the system. The EWOD control electronics generates, under control of the processor 3b, actuation voltages 5 for applying to element electrodes 38 (not shown) on lower substrate 36 and a common electrode (not shown) on top substrate 72 of the microfluidic device 2, for example to effect a pre-determined sequence of droplet operations. Fig. 3b shows the processor 3b as separate from the control electronics 3a, 3d and communicating with the control electronics 3a, 3d over a datalink 7, but the processor 3b and the control electronics 3a could alternatively be integral with one another. Fig. 3b also shows the EWOD control electronics 3a as separate from the EWOD device 2 and communicating with the EWOD device via an electrical connector 8, but the EWOD control electronics 3a and the EWOD device 2 could alternatively be integral with one another. The control electronics may include suitable circuitry and/or processing devices that are configured to carry out various control operations relating to control of the EWOD device, such as a CPU, microcontroller or microprocessor.

The sensing control electronics 3d receive sensor data signals 9 from one or more sensors (not shown) of the EWOD device 2. The sensor data signals 9 may include signals obtained by operating the EWOD array elements in a sensing mode, and/or may include signals obtained by one or more sensors external to the EWOD array elements such as some or all of illumination and/or detection optics, a thermal control unit or magnetic systems for interacting with the droplets 4. The sensor output of EWOD 2 is initially received by differential current sensor 200, which is configured to reduce the effects of any interfering signals that might be transmitted from the EWOD device 2. The sensor output is subsequently processed by voltage amplifier 300, to yield data signal 9, which pass via sensing control electronics 3d back to the processor 3b, which is configured to display the results on a user interface and store the results in a storage device (not shown).

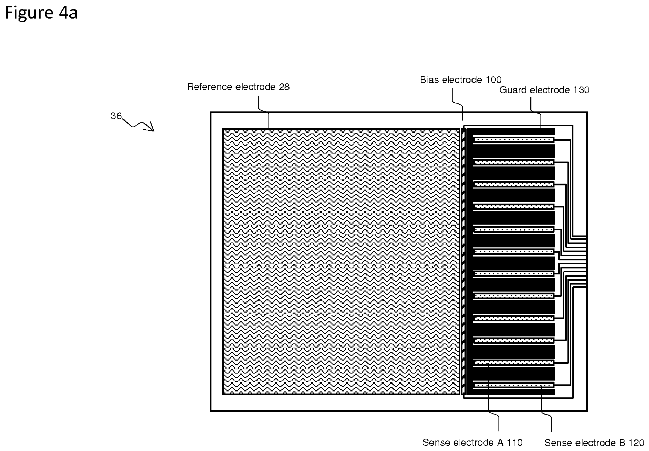

Fig. 4a represents a plan view of an exemplary embodiment of an improved top substrate 36 that may be used with the EWOD device of Fig. 1 to achieve high sensitivity measurement of currents associated with droplets, either arranged with DIB interfaces therebetween, or for performing direct electrochemical measurement of a component of the fluid within a droplet, which droplets are being manipulated within the EWOD device. Fig. 4a comprises a reference electrode 28, in common with the device of Fig. 1; however, Fig. 4a further comprises a series of discretely defined electrodes, which may be addressed in common with the reference electrode 28 and so serve as an extension of the reference electrode 28 in one instance, but may alternatively serve as independently addressable electrodes, including bias electrode 100, sense electrode A 110, sense electrode B 120 and guard electrode 130. As will be understood from the description below, a sense electrode A 110 is intended for use with a control droplet or control droplet pair, whereas a sense electrode B 120 is intended for use with a droplet or droplet pair whose characteristic is to be measured or monitored. Whether a sense electrode is a sense electrode A 110 or a sense electrode B 120 is to some extent determined in use, and in principle all the sense electrodes could be identical to one another. In some cases however the optimum geometries of an electrode and/or the wiring connecting to it may differ between a sense electrode A 110 (or "control electrode") and a sense electrode B 120, (for example the sense electrode may be smaller to minimise cross talk to it) - in these cases it may be preferable to designate a sense electrode as either a sense electrode A 110 or a sense electrode B 120 during design/manufacture of the EWOD device and provide the appropriate geometry for the electrode and/or the connecting wiring. The substrate 36 may for example be manufactured by disposing a conductive layer over the entire area (or entire active area) of the substrate, and patterning the conductive layer to form the reference electrode 28 and the further independently addressable electrodes 100, 110, 120, 130. Non-limiting examples of suitable materials for the conductive layer include, but are not limited to Indium Tin Oxide (ITO), Indium Zinc Oxide (IZO), Gold, Silver, Platinum, Aluminium, or poly(3,4-ethylenedioxythiophene) polystyrene sulfonate (PEDOT:PSS).

Within the configuration of Fig. 4a, a sensing channel is defined by sense electrode A 110, sense electrode B 120 and the bias electrode 100. Fig. 4a shows multiple sensing channels, although in principle only one, or any number as may be required, sensing channel(s) need be provided. In the embodiment of Fig. 4a bias electrode 100 is common to all sensing channels, however different sensing channels could have separate bias electrodes if required. Guard electrode 130 is provided to mitigate potential inductance affects that may otherwise compromise the integrity of measurement signals made using respective sense electrodes A 110 and sense electrodes B 120, when paired with bias electrode 100.

During electrowetting manipulation of droplets within the EWOD device, reference electrode 28 and bias electrode 100, sense electrode A 110, sense electrode B 120 and guard electrode 130 may all be biased to act as a single reference electrode 28 against element electrodes 38A and 38B on lower substrate 72 (as depicted in Fig. 1). However, in some aspects of the invention, movement of droplets within the EWOD device, when such droplets are positioned beneath the region of top plate 36 that is patterned with bias electrode 100, sense electrode A 110, sense electrode B 120 and guard electrode 130, may be achieved solely through potentials applied through array element electrodes (38A, 38B), without requiring a path via reference electrode 28.

In an exemplary embodiment of the invention, bias electrode 100, a sense electrode A 110, and a sense electrode B 120 are used to sense a property or characteristic of a droplet, or to monitor a change resulting from transfer of a solute or molecule of interest into or out of the droplet. Measurements may be made between a respective sense electrode A 110 or sense electrode B 120 and the bias electrode 100. As such, sense electrode A 110 or sense electrode B 120 may act as a cathode against bias electrode 100 acting as anode (or vice versa). Thus, in an exemplary embodiment, a droplet may be located to be in electrical contact with bias electrode 100 and sense electrode A 110 or bias electrode 100 and sense electrode B 120 respectively. A voltage may be applied between the bias electrode 100 and the sense electrode A 110 or sense electrode B 120, which results in a current flow through the droplet in contact therewith. The current may be monitored over time to obtain information about a process occurring in the droplet.

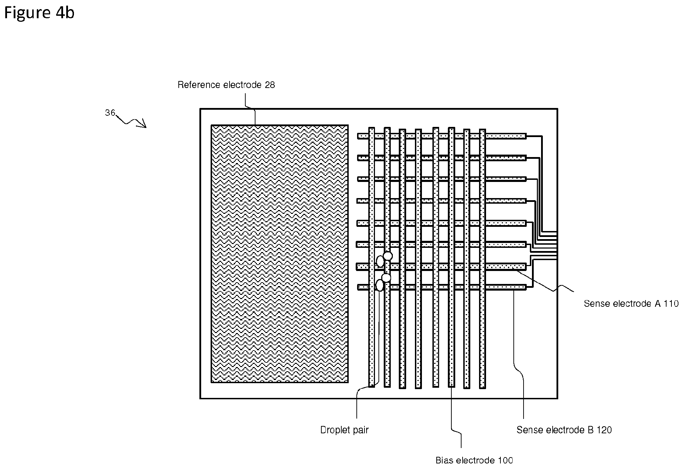

Fig. 4b depicts another embodiment of an improved top substrate 36. In this configuration, during the manufacturing process, a first conductive layer is applied to the substrate, which is patterned to define pairs of sense electrodes A and B as horizontal bars. An insulation layer applied over the first conductive layer ensures that the two layers of electrodes are not directly connected at the crossover points. A further conductive layer is applied, which is patterned to define vertical bars that represent bias electrode 100. The insulator layer is then removed in the region of the crossover points to ensure both electrode layers can make direct electrical contact to any droplet brought in contact therewith. Two associated droplet pairs are shown, each connected to a common bias electrode and to different sense electrodes.

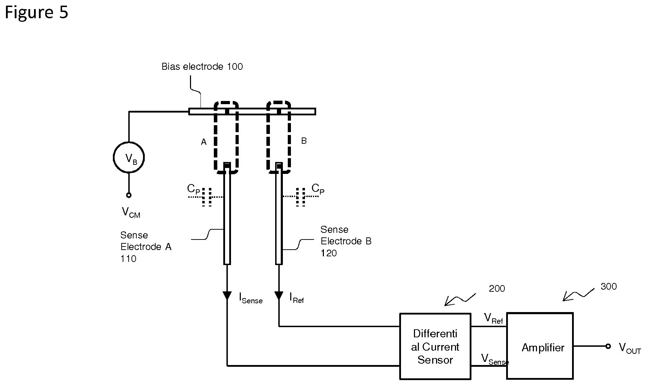

Two (or in principle more) droplets may be positioned so as to each contact a respective bias electrode and a respective sense electrodes, and with selective application of a potential to the relevant rows and columns, individual droplets may be monitored at the intersections between respective bias electrodes 100 and sense electrodes 110, 120. The sensing arrangement is then as Fig. 5.

The electrode arrangement of Fig. 4b facilitates a larger number of associated droplet pairs to be sensed than would be the case for the electrode arrangement of Fig. 4a. For the arrangement of Fig. 4a, the total number of electrodes required to sense N droplet pairs scales as ~N+1. In the arrangement of Fig. 4b it scales as ~ sqrt(N). Against this, however, the method of construction of the embodiment of Fig 4b represents a more costly process compared with the embodiment of Fig. 4a, due to the requirements for multiple applied layers.

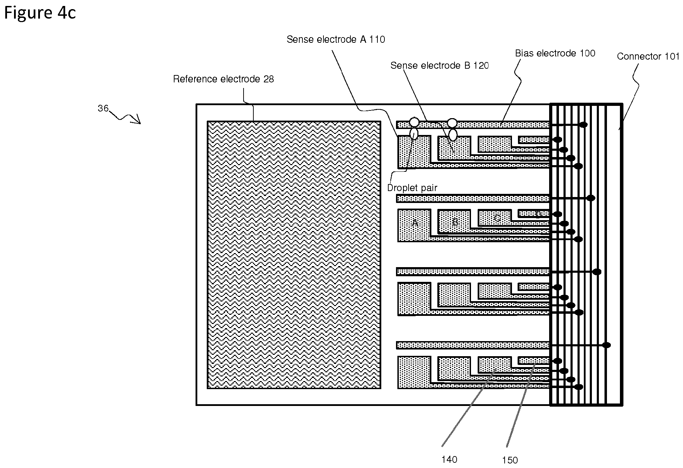

Fig. 4c represents a further embodiment of an improved top substrate 36. In the configuration of Fig. 4c a single conductive layer is applied to the substrate, which conductive layer is pattered to define reference electrode 28 and respective sets of bias electrodes 100 and sense electrodes A 110, B 120, C 140, D 150. Electrical connection is made to the respective bias and sense electrodes using an external connector 101. In the configuration of Fig. 4c (as depicted) measurements may be made simultaneously on respective groups of electrodes A, B, C, D by the selective application of a voltage to each of A, B, C or D and measurement of the associated current sourced (or sunk) through each of A, B, C, D individually. A bias electrode 100 is common to each group of four sense electrodes, but a bias electrode for one group of four sense electrodes A-D does not share a common conductor on connector 101 with a bias electrode for another group of four sense electrodes A-D. Thus, although respective sense electrodes A, for example, share a common conductor on connector 101, because each sense electrode A is associated with a distinct bias electrode 100, distinct circuits are formed for each respective sense electrode, permitting multiple measurements to be made in parallel. Other configurations of electrode groups may be achieved, however adding further sense electrodes would require a more densely packed series of conductors on the connector 101, which may render the device more vulnerable to parasitic capacitance effects.

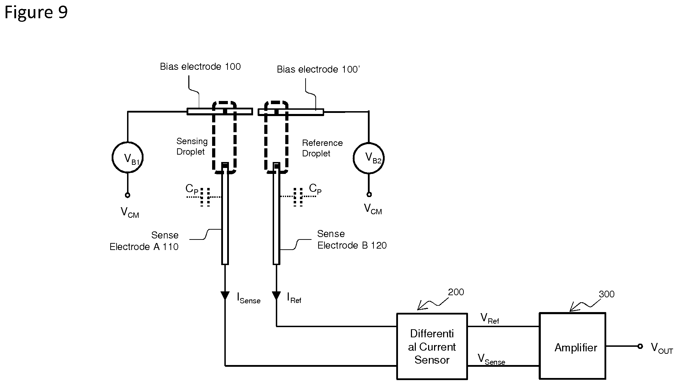

When making pair wise measurements using a top plate as patterned according to Fig. 4a, for example, it may be desirable to make comparative measurements using respective sense electrodes A or B that have generally similar conductive path lengths, in order to minimise influences of parasitic capacitance or electromagnetic interference. Fig. 9 shows two droplet pairs, one pair making contact with the bias electrode 100 and a sense electrode A 110, and the other pair making contact with the bias electrode 100 and a sense electrode B 120. The droplet pair making contact with the bias electrode 100 and a sense electrode B 120 is a reference droplet pair, in that the expected behaviour with time of the current $I_{Ref}$ flowing through the reference droplet pair is known, and may be used to determine whether a change in the current $I_{Sense}$ flowing through the other droplet pair (in contact with bias electrode 100 and sense electrode A 110) is a result of a change in the properties of the droplet or is an artefact of the measurement process. Conveniently, the reference droplet pair may be arranged such that its properties are not expected to vary over time so that $I_{Ref}$ is expected to stay constant over time.

Guard electrode 130 is preferably provided to minimise effects of parasitic coupling between respective sense electrodes A 110 and sense electrodes B 120. As the relative density i.e. the total number increases, and respective width and gap distance between them decreases, sense electrodes A 110 and sense electrodes B 120, become increasingly more susceptible parasitic capacitance. That is, when two conductors are sufficiently close, and carrying different voltages, then charge coupling effects can occur between respective tracks. Guard electrode 130, which may either be connected to ground or to a DC circuit of electrode control electronics 3a, serves as a shield mitigating undesirable charging effects in either sense electrode A 110 or sense electrode B 120.

The electrode structure of Fig. 4a may also be used with a single droplet rather than a droplet pair, as shown in Fig. 5 for example. Again, the droplet making contact with the bias electrode 100 and a sense electrode B 120 is a reference droplet, in that the expected behaviour with time of the current $I_{Ref}$ flowing through the reference droplet is known (for example is expected to stay constant).

Figs. 5 and 7a to 12 represent exemplary circuit configurations according to a series of embodiments of the invention that may be used to monitor a process in a fluid package (that is, within a droplet) such as of solute transfer in a droplet pair through a transporter species, or electrochemical reaction within a droplet. In traditional measurement configurations it has been observed that electrical interference, such as EMI and other interferences, including parasitic capacitance, may adversely affect measurement of small currents in the range of pA, when a species of interest is transferred across a DIB.

In accordance with a first circuit embodiment, Fig. 5 depicts an arrangement where two droplets A and B (shown as elongated droplets for clarity in Fig. 5, though they don't necessarily need to have the shape shown in Fig. 5) are provided in contact with bias electrode 100 and in contact with sense electrode A 110 or sense electrode B 120 respectively. A bias voltage $V_B$ is applied between bias electrode 100 and sense electrode A 110 or sense electrode B 120 respectively, which results in currents $I_{Sense}$ and $I_{Ref}$. It is desired to measure the currents $I_{Sense}$ through droplet A, to provide information about an electrical property of droplet A such as its conductance. If, for example, a reaction that is occurring in droplet A has the effect of changing the conductivity, measuring the currents $I_{Sense}$ over a period of time provides information on how the conductance of the droplet varies over time and hence provides information on the progress of the reaction responsible for the change in conductance. Both sense electrode A 110 and sense electrode B 120 may be susceptible to noise / cross talk via parasitic capacitance represented by capacitor $C_p$. Generally, the measurement value $I_{Sense}$ of Droplet A represents the parameter of interest and the measurement value $I_{Ref}$ of Droplet B represents a reference signal onto which interference or noise will couple in a similar manner as onto $I_{Sense}$. By differential sensing, eg sensing the difference between the two currents ($I_{Sense}$ - $I_{Ref}$), the interference or noise may be subtracted from the measured $I_{Sense}$. The two values $I_{Sense}$ and $I_{Ref}$ are fed into a differential sensing circuit, which processes the respective values to yield an output $V_{OUT}$ that represents the difference between $I_{Sense}$ and $I_{Ref}$ and comprises a truer measurement of the signal of interest substantially free from effects of interference or noise. $V_B$ may be supplied as either a constant magnitude DC voltage or an AC voltage of constant peak-peak magnitude, depending on the intended purpose of the sensor circuit. When $V_B$ is provided as an AC voltage, the frequency with which values are sampled by the differential current sensor (DCS) 200 is lower, typically an order of magnitude lower, than the AC frequency. The DCS receives both $I_{Sense}$ and $I_{Ref}$ as current values and converts them to an output voltage value, that is substantially devoid of noise / crosstalk. When constructing the circuit, the skilled artisan will recognise the desirability of using conductive pathways that are essentially equivalent in terms of path length, width and conductivity, between paired circuits, such that parasitic capacitance and EMI affect each circuit similarly.

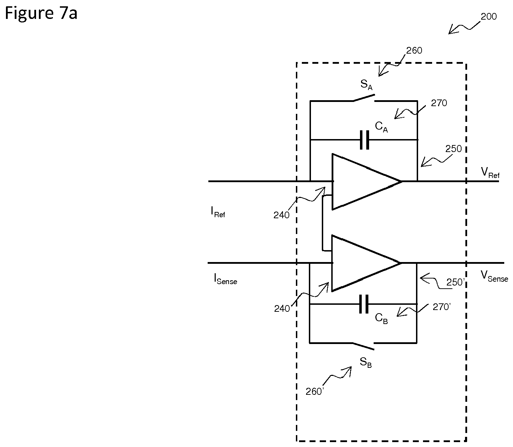

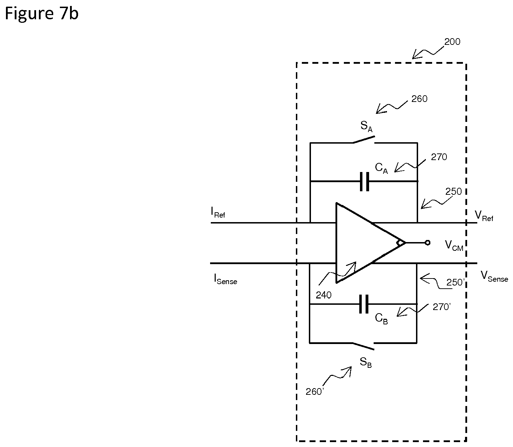

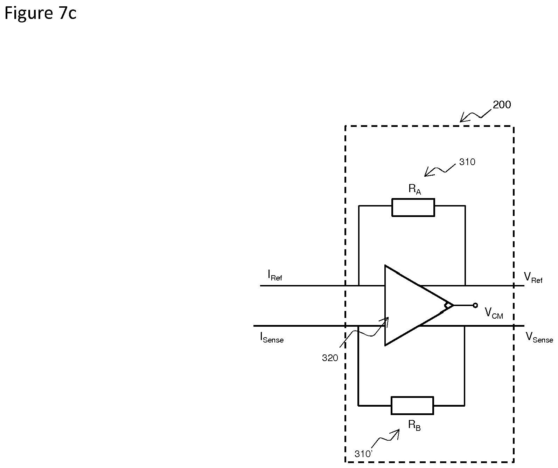

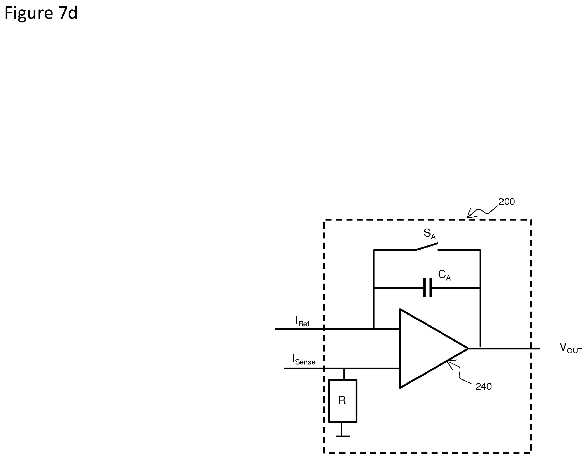

The present embodiment is an exemplary arrangement for determining the differential output $I_{Sense}$ - $I_{Ref}$. The differential current sensor 200 may be of a standard arrangement comprising components as will be described in more details with reference to Figs. 7a-d. As such, DCS 200 may be selected from any one of those depicted in Figs. 7 a to d (or functional equivalents thereof as will be apparent to the person skilled in the art). The DCS 200 processes the input signal in such a way that the noise is essentially cancelled out, thus improving the signal to noise ratio. An amplifier 300 receives the voltage output from the DCS, and further process the signal to yield a voltage of appropriate magnitude to be recorded by a suitable recorder (not shown). Amplifier 300 produces voltage $V_{OUT}$, which is transferred to sensing control electronics 3d (as depicted in Fig. 3b), which voltage data is used to prepare a profile of signal over time, which is representative of the process occurring within the droplet of interest.

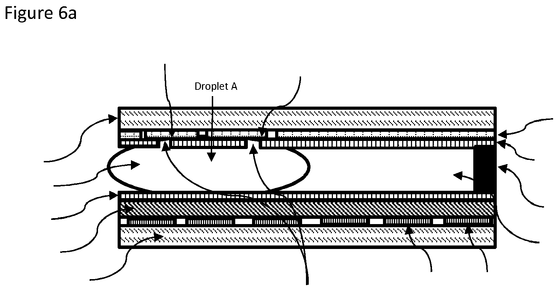

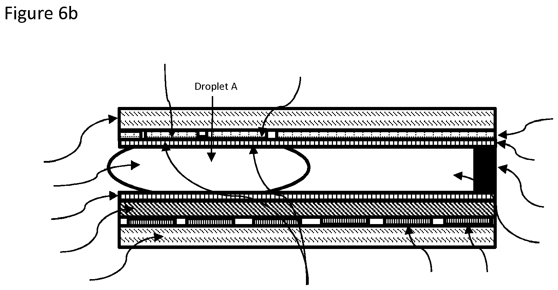

Figs. 6a and b represent cross sectional views through a single droplet as depicted in Fig. 5. Fig. 6a shows a single Droplet A positioned beneath sense electrode A 110 and sense electrode B 120; in which apertures are provided in hydrophobic coating 26, thereby forming pathways for direct contact between sense electrode A 110 and sense electrode B 120 and droplet A. In such a configuration, an electrochemical process within Droplet A may be monitored by applying a voltage between sense electrode A 110 and sense electrode B 120, and monitoring the resulting current that flows at one or the other electrode. In this embodiment, one of the sense electrodes acts as the bias electrode of other embodiments - rather than applying a voltage via the bias electrode 100 as in figure 5, one of the sense electrodes is used to apply a bias voltage to the droplet.

Fig. 6b depicts an alternate configuration to the cross sectional view of Fig. 6a, in which hydrophobic coating 26 is contiguous across the surface of sense electrode A 110 and sense electrode B 120, thus providing a barrier layer between Droplet A and the respective sense electrodes 110, 120. The effect of hydrophobic coating on the electrode surface means that Droplet A is electrically coupled by capacitive means to the respective sense electrodes 110, 120. Advantageously, maintaining a continuous hydrophobic coating avoids the need to pattern the layer in this area, thus making the device simpler to manufacture. Furthermore, the presence of hydrophobic coating 26 over sense electrodes 110, 120 is such that effects such as hydrolysis of the polar fluid may be minimised when DC voltages exceeding 1.23V are applied between bias electrode 100 and sense electrode A 110 or sense electrode B 120.

When measuring current responses within EWOD devices, for example, where currents may typically be in the picoampere range, there is a risk of effects such as electromagnetic interference, coupled, for example, through parasitic capacitance onto the conductive pathways that connect sense electrode A 110 and sense electrode B 120 to a microprocessor configured to determine a measurement value within the electrode control electronics 3a (as depicted in Fig. 3a; and as discussed with reference to Figs. 5 and 7a to 12). Aspects of the present invention seeks to mitigate such interferences through use of selective sensor circuit configurations based on differential current sensors, which incorporate features designed to minimise the influence of "noise" and therefore achieve an increased signal-to-noise, improving the likelihood of success of very sensitive measurements.

Fig. 7a depicts a first circuit configuration of a DCS 200. The circuit comprises a pair of operational amplifiers (op-amps) configured in single ended mode, which comprise op-amps 240, 240', configured to receive current inputs $I_{Sense}$ and $I_{Ref}$ respectively produced from Droplets A and B; with each op-amp 240, 240' outputting voltages $V_{Sense}$ and $V_{Ref}$, which are fed into amplifier 300, which yields a single output $V_{OUT}$ that is transferred to sensing control electronics 3d.

Fig. 7b depicts a second circuit configuration of a DCS 200. The circuit comprises an operational amplifier 240 configured in differential mode, switches 260, 260', and capacitors 270, 270'. Op-amp 240 receives current inputs $I_{Sense}$ and $I_{Ref}$, which are referenced against the common mode voltage $V_{CM}$. The DCS 200 samples signals from $I_{Sense}$ and $I_{Ref}$ and processes the signals to yield a single output $V_{OUT}$. The feedback loops 250, 250' include switches 260, 260' which when closed reset the charge stored in the capacitor 270, 270', which results in the output $V_{OUT}$ also being reset. The sampling rate of switches 260, 260' determines the frequency at which the input signal is sampled to create an output $V_{OUT}$.

Fig. 7c, represents a different exemplary circuit arrangement employed to determine the differential output signal $I_{Sense}$ - $I_{Ref}$. The DCS of fig 7c is configured with a transimpedance amplifier (TIA) which comprises a differential amplifier 320, and resistors 310, 310'. In this configuration, when $V_B$ is DC the circuit will measure the difference in resistance of the sensing and reference droplets; and when $V_B$ is AC, the circuit will measure the real and imaginary droplet impedance, by measuring relative amplitude and phase change of AC currents $I_{Sense}$ and $I_{Ref}$. The DCS produces $V_{Sense}$ and $V_{Ref}$, which are further processed to yield $V_{OUT}$, which represents the desired signal due to the process of interest, substantially free from interference, similar to the circuits of Fig. 7a and 7b.

Fig. 7d represents yet another exemplary circuit arrangement employed to determine the differential output signal $I_{Sense}$ - $I_{Ref}$. In the configurations of Fig. 6d, $I_{Sense}$ is connected via a resistor R to ground, such that the first stage of the sensing circuit is configured as a single ended integrator. The circuit of Fig. 7d is expected to yield similar performance improvements as the circuit of Fig. 7b, however it may be realised with a standard single-ended integrator arrangement and as such may be more conveniently manufactured using commercially available components.

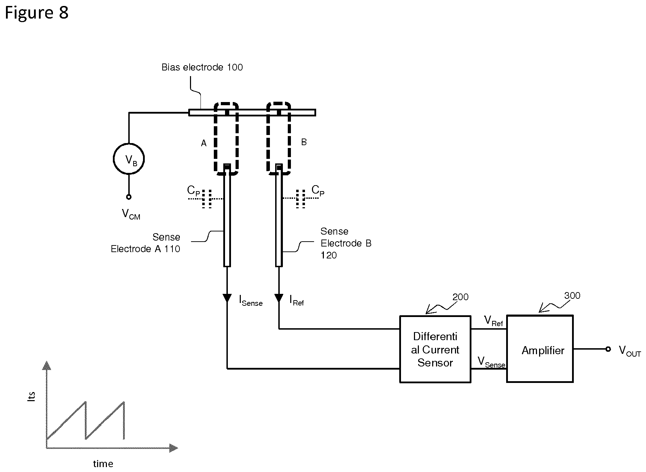

Fig. 8 is similar to Fig. 5, however in this embodiment $V_B$ is supplied as a saw tooth wave, which has a linearly increasing value, that sweeps from a minimum to a maximum value and then drops back to the starting value and repeats (as shown in the inset to Fig. 8). DCS 200 may be configured using any of the circuits depicted in Figs. 7a to d. During the process of changing the voltage value, DCS 200 may be used to measure different characteristics of the droplet when exposed to increasing voltage. When $V_B$ is supplied as an AC voltage, either the peak-to-peak amplitude or the frequency may be modulated with measurement values $V_{OUT}$ being recorded at different values of $V_B$.

Fig. 9 represents a further modification of the circuit described in Fig. 5, in which a second bias electrode 100' is included. One droplet makes contact with one bias electrode 100 and sense electrode A 110, and the other droplet makes contact with the second bias electrode 100' and sense electrode B 120, such that Droplet A and Droplet B may be exposed to different applied bias voltages, $V_{B1}$ and $V_{B2}$ respectively. At the start of a test procedure before a reaction process within Droplet A has been initiated, $V_{B1}$ and $V_{B2}$ may be modulated such that the initial values for $I_{Sense}$ and $I_{Ref}$ are essentially of equal value, thereby compensating for possible variation in the size or volume of the two droplets; or for other variances in the respective circuits with which each droplet is a part. Any measurements made of a reaction processes of interest occurring within Droplet A may therefore yield further refinement to the value of output $V_{OUT}$, since further variance due to differences caused by variation of the size, volume or composition, in addition to the effects of parasitic capacitance or electromagnetic interference that are compensated for by Droplet B, may be taken into consideration when generating $V_{OUT}$.

The embodiment of Fig. 9 (which comprises two independently addressable bias electrodes) may be applied with any suitable circuit arrangement employed to determine the differential output signal $I_{Sense} - I_{Ref}$, in which the DCS may be selected from one of those described with reference to Figs. 7a to d.

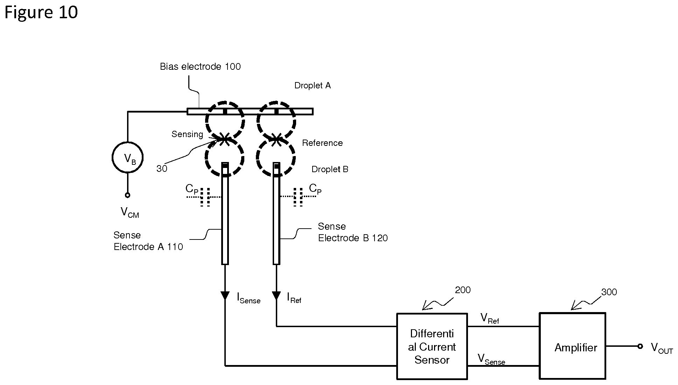

Figs. 10 to 12 represent exemplary circuit configurations according to a series of embodiments of the invention that may be used to monitor a process in pairs of droplets arranged such that there is a droplet interface bilayer (DIB) between them. As such processes of solute transfer from one droplet pair to the other through a transporter species (such as for example a nanopore) inserted in the DIB, or for monitoring an electrochemical reaction within one of the droplet pair.

Fig. 10 corresponds to the circuit of Fig.5, however unlike Fig. 5, two pairs of droplets are provided in place of the single elongate droplets. In one embodiment, the sensing pair of droplets may differ from the reference pair, in that the sensing pair may include a transporter species 30 within the DIB which gives rise to a change in measured current when a molecule of interest is transported through the transporter species. The reference pair of droplets may also form a DIB, but there may or may not be a transporter species present within the DIB. Both sets of Droplets A and B may contain similar solutions; however when the reference pair of droplets lacks the transporter species, signals arising from the reference pair may be subtracted from signals from the sensing pair to yield a signal indicative of the movement of the target of interest across the transporter species, which is substantially free from the effects of noise as described herein above. The bias voltage $V_B$ is typically a DC voltage, but could in principle be an AC voltage.

In an alternative configuration, both pairs of droplets A and B include an active transporter species within their respective DIBs, but fluid within Droplet A of the reference pair may lack the molecule of interest, that is present in droplet A of the sensing pair. Again, signals arising from the reference pair may be subtracted from signals arising from the sensing pair to yield a measurement value that is more representative of the movement of the molecule of interest across the DIB of the sensing pair via the transporter species, devoid of the influence of noise.

As was described with reference to Fig. 5, in the embodiment of Fig. 10 the DCS 200 receives current values $I_{Sense}$ and $I_{Ref}$ from sense electrode A 110 and sense electrode B 120 and produces an output value $V_{OUT}$, which is a more accurate representation of the active transfer process that occurs when a molecule of interest is transported via a transporter species 30 from Droplet A into Droplet B.

Any variation in current that occurs due to coupling via parasitic capacitance or electromagnetic interference, which may otherwise adversely change the current value that is measured when a molecule of interest is transported through a transporter species or diffuses across a DIB may thus be substantially eliminated through the use of the reference pair of droplets, which would be expected to experience essentially the same parasitic capacitance or electromagnetic interference as the sensing pair of droplets, within acceptable tolerance limits.

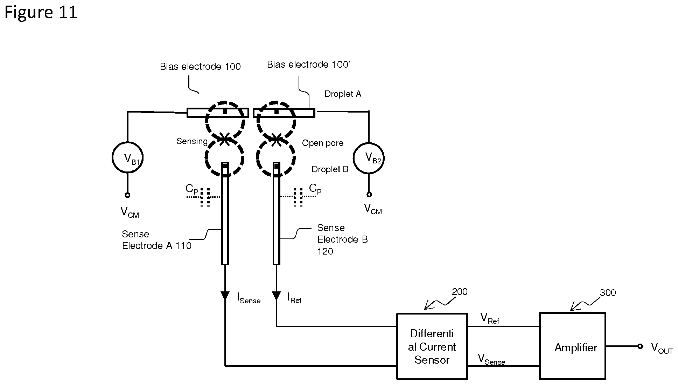

Fig. 11 corresponds to Fig. 10, but with the presence of two pairs of droplets, respectively having a DIB therebetween, in which respective Droplet A's are in contact with distinct bias electrodes 100, 100'. As described with reference to Fig. 9, the presence of two bias electrodes 100, 100' allows for independent modulation of $V_{B1}$ and $V_{B2}$ prior to commencement of a transfer process to compensate for any initial imbalance in respective values of $I_{Sense}$ and $I_{Ref}$, which would be expected to be equal. During the course of a measurement, for example the transport of a molecule of interest through a transporter species 30 present in the sensing pair of droplets, $V_{B1}$ may further be modulated up until the point at which the active transfer of the molecule of interest begins to occur, thereby ensuring any influences of noise prior to commencement of a measurement are minimised or compensated for. As such, making ongoing minor adjustments may further enhance the differences between the measured baseline signal and a measured signal of interest that arises from the sensing pair of droplets, leading to an improved signal to noise ratio. The processing of $I_{Sense}$ and $I_{Ref}$ through DCS 200 may further enhance the difference between signal and noise, thus improving the ability to discriminate low level signals, which may otherwise be lost in system noise.

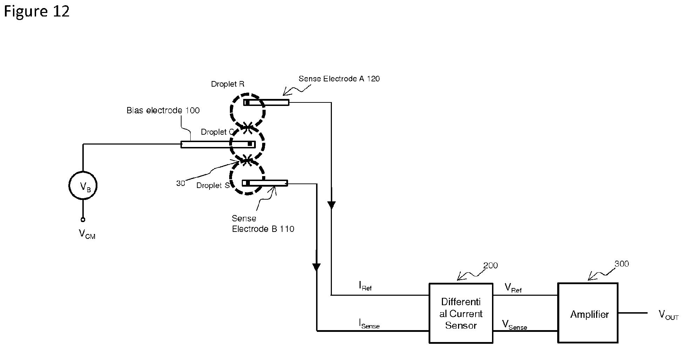

Fig. 12 represents an alternative configuration, in which three droplets are arranged such that a central "receiving" droplet, droplet C, is connected via DIBs to a sense droplet, droplet S, and a reference droplet, droplet R. This arrangement forms a divider, such that the currents developed at sense electrode A 110 and sense electrode B 120 depend on the ratio of (1) the resistance between sense electrode A 110 and bias electrode 100 and (2) the resistance between sense electrode B 120 and bias electrode 100. The simplified circuit is configured with a single bias electrode 100 and two sense electrodes, A 110 and B 120, which yield current output values $I_{Sense}$ and $I_{Ref}$ respectively, that are fed through DCS 200 to a voltage amplifier 300, which leads to $V_{OUT}$, as with the previous circuits. The DIB between sense droplet, droplet S, and receiving droplet, droplet C, contains a transporter species 30, whereas the DIB between reference droplet R and receiving droplet C may or may not contain a transporter species. If there is a transporter species in the DIB between reference droplet R and receiving droplet C then the reference droplet R lacks the molecule of interest; if the reference droplet R lacks a transporter species, then it may contain a molecule of interest, which would not be expected to migrate into receiving droplet C. $V_B$ is maintained to ensure current flows through the transporter species between sensing droplet S and receiving droplet C. It is expected that parasitic capacitance and electromagnetic interference will affect respective electrodes equally, and thus would not be expected to adversely affect the current, $I_{Sense}$, as described with reference to the previous embodiments shown in Figs. 5 to 10. The factors that perturb the measured currents $I_{Sense}$ and $I_{Ref}$ of the respective droplets are expected to affect both the sensing (droplet S) and reference (droplet R) droplets equally. In this embodiment (as with the other embodiments described herein above), whilst it is the current through the pore(s) that is sensed, it is read out as a voltage, $V_{OUT}$, once the current values have been processed by DCS 200 and amplifier 300.

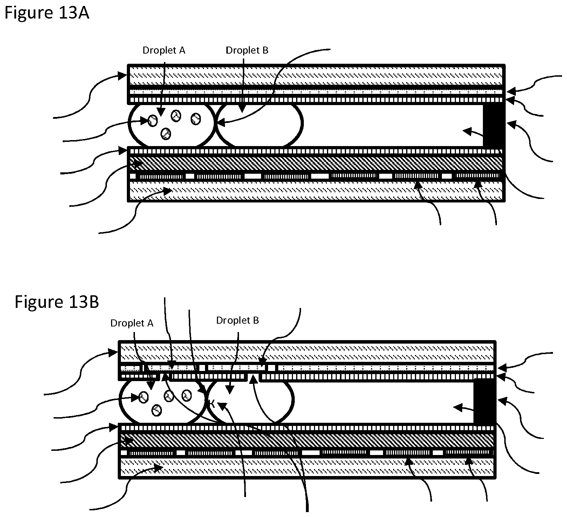

Fig. 13a-b illustrate a cross-sectional view of a droplet interface bilayer in a microfluidic device such as an AM-EWOD device. Initially, with reference to Fig. 13a and b, two fluid droplets, Droplet A, and Droplet B are shown. Typically, Droplet A and Droplet B are droplets of polar fluid and are disposed in a non-polar filler fluid 34 (such as oil) disposed in the fluid space of the EWOD device, as shown generally in Fig. 1.

As used herein, specifying that a first droplet and a second droplet "may be manipulated so as to be brought together by appropriate actuation of the array element electrodes of the EWOD device" covers a case where array element electrodes of the EWOD device are actuated to hold the first droplet stationary and move the second droplet into contact with the first droplet, a case where array element electrodes of the EWOD device are actuated to hold the second droplet stationary and move the first droplet into contact with the second droplet, and a case where array element electrodes of the EWOD device are actuated to move the first droplet and to move the second droplet so that the first and second droplets make contact with one another.

Droplet A contains fluid (e.g. liquid) of a first composition that includes at least a first solute species in a first solvent. In the case of polar fluid droplets, the first solvent is typically water although it could be another polar solvent such as methanol, for example. The second droplet, droplet B, contains fluid (e.g. liquid) of a second composition that may be the same or different to the composition of the fluid of droplet A. The fluid in droplet B may contain one or more solute species in a solvent, and the fluid of droplet B may have the same or a higher overall solute concentration than the fluid of droplet A. As used herein, a "solute" is a substance that is dissolved in another substance, known as a "solvent", to form a mixture known as a "solution".

The solvent in droplet B will generally be the same solvent as the solvent in droplet A, although the invention is in principle not limited to this. As a result, the solution in droplet B may have a higher osmotic pressure than droplet A. (In a case where the solvent (second solvent) in droplet B is not the same as the solvent (first solvent) in droplet A, it is possible that the DIB between droplet A and droplet B will be permeable to both the first solvent and the second solvent, so that the two resultant droplets include both the first solvent and the second solvent - if a droplet having methanol as its solvent and a droplet having water as its solvent were to form a droplet interface bilayer, as an example, one would expect that the DIB would be permeable to both water and methanol. After formation of the DIB, each droplet would therefore be likely to contain a mixture of the first and second solvents. The solute composition of the droplets will depend on the permeability of the DIB to the solute(s) present in the initial droplets.)

According to the embodiments of Fig. 13a-b, Droplet A and Droplet B are manipulated to come together in such a way that they do not merge into a single droplet, but instead form a droplet interface bilayer (DIB) on contact between the respective meniscus of each droplet. Each droplet is surrounded by a monolayer of amphiphilic molecules, such as a lipid monolayer. Amphiphilic molecules used to make up the DIB may be introduced from either the droplet (using the 'lipid in' method) or the filler fluid (using the 'lipid-out' method). The "lipid in" and "lipid out" methods are well known techniques for forming DIBs, and are described in, for example, "Sensors and Actuators B: Chemical", 2013, 185 pp530 to 542. A wide range of amphiphilic molecules can be used to form lipid monolayers; including lipids, surfactants and detergents examples of polymer surfactants are diblock and triblock copolymers (but the invention is not limited to these).

When the two droplets are manipulated, for example by electrowetting in an EWOD device, so as to come together, the amphiphilic molecules of one droplet interact with the amphiphilic molecules of another droplet so that the amphiphilic monolayers of the two droplets form a droplet interface bilayer 10 as shown in Fig. 13a. Formation of a droplet interface bilayer is a reversible process, and the two droplets may subsequently be manipulated to separate from one another - provided they have not merged into a single droplet.

The droplet interface bilayer 10 forms a semipermeable membrane, so that small molecules can pass from one droplet, through the droplet interface bilayer, into the second droplet whereas larger molecules generally cannot pass through the droplet interface bilayer 10. Provided that the molecules of the polar solvent in droplet A are small - for example if the solvent is water or methanol - solvent molecules can pass through the droplet interface bilayer. The difference in the osmotic pressure of the solutions in droplet A and droplet B results in the movement of solvent molecules from Droplet A across the droplet interface bilayer 10 into Droplet B.

The solute species in Droplet A and Droplet B are typically large molecules, charged molecules or ions, which may pass through the droplet interface bilayer, albeit they may pass through the DIB but at a much lower rate than the rate at which solvent molecules can pass through the DIB. For example, it is likely that a solute species comprising ions or highly charged molecules would not be able to pass through a DIB, whereas dye molecules that are neutral or have a small charge would probably pass through a DIB, but at a low rate. The transport properties of a DIB depend inter alia on the structure of the DIB, and which surfactants have been used in its formation.

The rate at which solvent passes from Droplet A to Droplet B across the droplet interface bilayer will depend inter alia on the difference in osmotic pressure between the fluid in Droplet A and the fluid in Droplet B when the DIB forms between Droplet A and Droplet B. The relative osmotic pressure of droplets A and B will depend very much on the specific application. In some applications, for example nucleic acid sequencing, the droplets will be arranged to have similar osmotic pressures, such that the relative volumes of droplets A and B are maintained as similar. In this application the solutions comprising droplets A and B may comprise a suitable redox couple such as ferri / ferro cyanide. For example, the difference in osmotic pressure between the fluid in Droplet A and the fluid in Droplet B is greater than approximately 5 kPa (equivalent to 1 mM sodium chloride solution) at room temperature.

As depicted in Fig. 13b, one of the droplets may contain a chemical species, or transmembrane protein, that assists in the transport of solvent molecules across the droplet interface bilayer 10. With reference to Fig. 13b, Droplet A contains a transporter species 30, which on formation of a DIB may be insertable into the DIB. Transporter species 30 may permit selective transfer of a solute molecule from Droplet A to Droplet B, to the exclusion of other solutes which may be present in Droplet A. One exemplary transmembrane protein is Aquaporin. Such species preferentially position themselves in a droplet interface bilayer and facilitate the transport of water molecules across the droplet interface bilayer. Aquaporins have been shown to insert into synthetic membranes and facilitate the transport of water through the membrane as described by Manish Kumar, Mariusz Grzelakowski, Julie Zilles, Mark Clark, and Wolfgang Meier, in Highly permeable polymeric membranes based on the incorporation of the functional water channel protein Aquaporin Z. Proceedings of the National Academy of Sciences, 2007, 104, 20719-20724. Other transmembrane proteins that will form pores for use in accordance with the invention can be derived from β-barrel pores or α-helix bundle pores. β-barrel pores comprise a barrel or channel that is formed from β-strands. Suitable β-barrel pores include, but are not limited to, β-toxins, such as α-hemolysin, anthrax toxin and leukocidins, and outer membrane proteins/porins of bacteria, such as *Mycobacterium smegmatis* porin (Msp), for example MspA, MspB, MspC or MspD, CsgG, outer membrane porin F (OmpF), outer membrane porin G (OmpG), outer membrane phospholipase A and *Neisseria* autotransporter lipoprotein (NalP) and other pores, such as lysenin. α-helix bundle pores comprise a barrel or channel that is formed from α-helices. Suitable α-helix bundle pores include, but are not limited to, inner membrane proteins and a outer membrane proteins, such as WZA and ClyA toxin. Other exemplary transmembrane proteins, which may be caused to form pores in a droplet interface bilayer include the following: pores based on Msp, α-hemolysin (a-HL), lysenin, CsgG, ClyA, Sp1 and haemolytic protein fragaceatoxin C (FraC). The transmembrane protein pore is preferably derived from CsgG, more preferably from CsgG from *E. coli Str.* K-12 substr. MC4100.

Another exemplary transmembrane protein is α-hemolysin, which has been used to form pores, such as nanopores, in droplet interface bilayers, as has been documented in the literature, e.g. Jetha N, Wiggin M and Marziali A "Forming an alpha-hemolysin nanopore for single-molecule analysis". Methods Mol Biol. 2009; 544:113-127. Jetha et al describe the use of α-hemolysin nanopores to perform single molecule analysis of DNA or RNA. As the nucleic acid is translocated through a nanopore, a measurement of the change in current that occurs across the DIB interface when each base passes through the nanopore is made.

Once a desired pore forming species has been selected, the size of Droplet A or Droplet B may be monitored over time, for example using an EWOD device impedance sensor or other sensor (such as described in Applicant's patent US 8,173,000, the contents of which are incorporated herein by reference+), until it is observed that either droplet has stopped changing in size, which indicates that the transfer of solvent from one droplet to another has ceased. If the initial compositions of Droplet A and Droplet B are known, the final compositions of the two droplets once the overall solute concentrations of the two droplets have become equal can be calculated.

Alternatively, the concentration of a particular species within Droplet A or Droplet B could be measured using a range of methods e.g. electrochemical, optical (absorption and fluorescence) measurements etc. In such cases the new composition of Droplet A and/or Droplet B may be determined even if the transfer of solvent across the droplet interface bilayer is stopped (by breaking the DIB and separating the droplets) before the overall solute concentrations of the two droplets have become equal.

According to the embodiment of Fig. 13a, Droplet A and Droplet B are arranged to form a DIB within an EWOD device that has a single reference electrode 28 and a plurality of array element electrodes 38A and 38B. Hydrophobic coating 26 is uniformly present over reference electrode 28. Therefore, Droplets A and B are essentially electrically insulated from reference electrode 28. In such a configuration, monitoring transfer of solute from Droplet A to Droplet B based on changes in an electrical characteristic, may not be feasible. However, changes in the respective size of either droplet may be monitored using either capacitance or impedance feedback from the integrated EWOD sensor (as for example described in Applicant's patent US 10,078,986, the contents of which are incorporated herein by reference). In this embodiment, one of the sense electrodes acts as the bias electrode of other embodiments - rather than applying a voltage via the bias electrode 100 as in figure 10 or 11, one of the sense electrodes is used to apply a bias voltage to the droplet pair.

Fig. 13b is similar to the view of Fig. 13a, with the exception that independently addressable electrodes have been patterned on the top substrate 36. One electrode pair, constituted by sense electrode A 110 and sense electrode B 120 are depicted, although it will be appreciated additional pairs of electrodes may be provided on top substrate 36 as required. In this embodiment, one of the sense electrodes acts as the bias electrode of other embodiments - rather than applying a voltage via the bias electrode 100 as in figure 10 or 11, one of the sense electrodes is used to apply a bias voltage to the droplet pair.

In addition to the definition of independently addressable electrodes 110, 120, apertures 200 are also formed in hydrophobic layer 26. Apertures 200 are dimensioned such that, to a droplet positioned thereunder, the apertures are insignificant in respect of general electrowetting effects (i.e. do not significantly impede droplet motion); however, apertures 200 are of sufficient dimension [from about 1 um to about 100um] to permit direct contact to be made with either Droplet A or Droplet B and sense electrode A 110 or sense electrode B 120 respectively, when a droplet is positioned directly beneath an aperture 200. A typical diameter of aperture that does not significantly impede droplet motion would be around <50% of the diameter of a droplet being manipulated over it. Thus, for a device intended to operate with small droplets (d=100um), the diameter of an aperture 200 should be 50um or lower, while for a device intended to operate with larger droplets (d=1000um) the diameter of an aperture 200 could be 500um or lower.

Sense electrode A 110 and sense electrode B 120 may be operated independently of the electrowetting electrodes 38A, 38B, although if required they may be configured to function as one with reference electrode 28 (as described herein above). When a voltage is applied between sense electrode A 110 and sense electrode B 120, the resulting current that flows across DIB 10 may be monitored. Such current may for example be due to the movement of molecules of interest 18 from Droplet A into Droplet B by a process of osmosis or electro-osmosis. Alternatively, in an embodiment where a transporter species 30 (such as for example a nanopore) exists within the DIB interface, the current that flows may be due to the active transport of a molecule of interest 18 through transporter species 30. For example, when a DNA molecule passes through a nanopore the magnitude of the current density across the nanopore surface depends inter alia on the composition of DNA occupying the nanopore. This makes it possible to sequence a DNA molecule by passing it though a nanopore and measuring the change in current as each base passes through the nanopore, each base giving rise to a different current because of the different number of charged groups present on each base.

In embodiments in which a bias voltage is applied to a droplet, or to a droplet pair, using a bias electrode 100 rather than a sense electrode, an aperture may again be formed in the hydrophobic layer to permit direct contact to be made between the bias electrode and the droplet or a droplet of the droplet pair. Again, such aperture is preferably dimensioned such that, to a droplet positioned thereunder, the aperture is insignificant in respect of general electrowetting effects (i.e. do not significantly impede droplet motion), but are of sufficient dimension [from about 1um to about 100um] to permit direct contact to be made with a droplet is positioned directly beneath the aperture.

Exemplary application of the improved measurement circuits of the invention to a method of measuring the properties of one or more droplets, will be described herein below.

Biochemical analyses

When a biochemical or chemical workflow is being performed through the manipulation of aqueous droplets in an EWOD device, it is often desired to measure properties of the fluid composition of a droplet in order to determine whether or to what extent a reaction has occurred. Examples of droplet measurement/detection techniques include optical measurements such as absorption or fluorescence, electrochemical measurements, such as determination of the pH, $pO_2$, or $pCO_2$ of the fluid; determination of the redox potential of a component of the fluid; measurement of the electrical conductivity of the droplet (which provides a measure of the ionic content of the droplet), or measurement of the speed of movement induced in a droplet for a given actuation voltage. These and other electrical detection techniques may be made more reliable if the determination of the fluid composition of the droplet is determined using an improved circuit of the invention before the measurement is made. The measurement of currents produced due to electrochemical oxidation of a solute within the fluid, in which a species of interest may be oxidised at the surface of sense electrode A 110. For example, the oxidation of glucose catalysed by glucose oxidase or glucose dehydrogenase may be monitored due to the ultimate oxidation of a reduced mediator species at the surface of sense electrode A 110. Non-limiting examples of such mediator species include ferrocene, ferricyanide, hydrogen peroxide, as are well known in the art, for example as described in Chen et al., RSC Advances, 2013, 3, 4473.

In another process, a change in droplet composition may be monitored during the process of transfer of a molecule of interest or a solute species from Droplet A to Droplet B. For example, the process may be to determine whether, and if so to what extent, the transfer of the molecule of interest or solute species has occurred. Examples of processes that might benefit from monitoring the transport of a species across the droplet interface bilayer include, without limitation:

- change of the pH of one or both droplets may be measured by a pH sensor provided in/on the EWOD device;
- change in the electrical conductivity of one or more droplets may be measured, for example using the array element electrodes 38A, 38B and the common electrode 28, or using sense electrode A 110 and sense electrode B 120;
- change in one or more optical properties of the droplet may be measured, such as absorption or fluorescence;
- change in the viscosity of one or both droplets may be measured, to detect changes arising from the transfer of the solute species from one droplet to another; such changes may be determined as a change in impedance of a droplet or change in the ability to move the droplet by EWOD;
- sensing changes in the droplet sizes arising during the process of transfer of the solute species from Droplet A to Droplet B. Any change in droplet size will depend on the permeability of the lipid bilayer and the osmotic pressures of the solutions of the droplets.

Nanopore sensing

Another application of the circuits of the invention as described herein may be applied to sequencing of nucleic acids in a range of sample matrices, including for example cell lysates to determine presence of absence of a particular microorganism, or to determine whether a clinical sample contains a particular sequence, which may aid in diagnosis of cancer or other indication, for example, in which subtle alterations in the nucleotide sequence is potentially indicative of a change of state. Nucleotide sequencing may also be part of a wider program to study genomic variation within a population, to aid better understanding of the benefits that may be conferred by specific sequences. With reference to the embodiment of Fig. 10, a protein or synthetic nanopore 30 may be inserted into a Droplet Interface Bilayer (DIB). This modifies the electrical characteristics of the DIB such that a current may flow through the pores. Typically the current through an "open" (i.e. no biomolecule within it) pore is ~100pA. A nanopore may be configured to sense / sequence DNA, as is very well known, for example as described in US5795782. By applying a DC potential across the nanopore, current flows though the pore. If a nucleic acid strand enters the pore, the current is modulated, with the pattern of modulation dependent on the nucleotide base passing through the pore at that time.

The arrangement of Fig. 10 could be used to perform sequencing as follows. A sensing channel is configured as conventional, generating an output current $I_{Sense}$. Two droplets, one of which contains DNA to be sequenced, are manipulated to come together and form a DIB. The voltage applied across the DIB $V_B$ is typically of order a few hundred milliVolts and $I_{Sense}$ is a function of nucleotide base present in the pore at the time of sampling. The DNA molecules pass through the pore, causing a change in current which is proportional to the respective base (A, G, C, T) as it passes through the pore. By monitoring a change in the current as the nucleotide passes through the nanopore, a signal is recorded that is dependent on the shape, size and length of the DNA sequence under investigation. The time for a nucleotide base to pass through the pore is typically on the order of 1ms, and so $I_{Sense}$ will be sampled at frequencies of typically around 10kHz. In this arrangement, the sensing channel is combined with a reference channel. The reference channel includes two droplets which are manipulated to come together and form a DIB into which a pore is inserted. However, neither droplet of the reference channel includes the DNA to be sequenced so there are no nucleotide bases present to pass through the pore. This pore therefore remains open and so the current passed $I_{Ref}$ is essentially constant to first order, and any variation that occurs in $I_{Ref}$ may be attributed to noise. $I_{Sense} - I_{Ref}$ is measured by DCS 200 and amplifier 300. The purpose of the differential arrangement is that any crosstalk or noise is expected to influence both the sensing pair of droplets and the reference pair of droplets equally and thus when the outputs of the sensing and reference channels are processed by the differential measurement arrangement, the influences of noise will be significantly reduced, thereby yielding a more accurate representation of the signal associated with a specific base. This facilitates a higher fidelity of sequencing current to be determined than is possible in a single-ended arrangement. Utilisation of the circuits of the invention may therefore facilitate a higher accuracy of nucleotide base calling, and provide a reduction in errors when determining a nucleic acid sequence.

## Claims

1. A method of determining a characteristic of a first pair of droplets in an electrowetting on dielectric microfluidic device substantially free from the influences of interference, comprising;

   forming a droplet interface bilayer between a first pair of droplets, a droplet of the first pair of droplets contacting a first sensing electrode and another droplet of the first pair of droplets contacting a first bias electrode;

   forming a droplet interface bilayer between a second pair of droplets, a droplet of the second pair of droplets contacting a second sensing electrode and another droplet of the second pair of droplets contacting a second bias electrode;

   inserting a transporter species into the droplet interface bilayer of the first pair of droplets;

   measuring a first signal between the first bias electrode and the first sensing electrode due to transport of a target species through the transporter species of the first pair of droplets and measuring a first signal between the second bias electrode and the second sensing electrode for the second pair of droplets;

   determining a difference between the first measured signal from the first pair of droplets and the first measured signal from the second pair of droplets; and

   reporting the difference between the first measured signal from the first pair of droplets and the first measured signal from the second pair of droplets as being the characteristic of the first pair of droplets, substantially free from interference.

2. The method of claim 1, wherein the measured first signal of the first pair of droplets comprises a signal component of interest and a first signal component of interference; and wherein the first signal of the second pair of droplets comprises a second component of interference.

3. The method of claim 2, wherein the signal component of interest in the measured first signal of the first pair of droplets is indicative of passage of a first species through the transporter species.

4. The method of claim 3 and further comprising inserting a nanopore into the droplet interface bilayer of the second pair of droplets, wherein the first species is absent from the second pair of droplets.

5. The method of claim 1, 2 or 3 wherein the first pair of droplets and the second pair of droplets have a droplet in common.

6. The method of claim 1, 2, 3 or 4 wherein the first bias electrode is continuous with the second bias electrode.

7. The method of claims 1 to 6, comprising transferring the first measured signal from the first pair of droplets and the first measured signal from the second pair of droplets to a signal processor configured to determine the difference between the first measured signal from the first pair of droplets and the first measured signal from the second pair of droplets.

8. A method as claimed in any preceding claim, wherein forming the droplet interface bilayer between the first pair of droplets comprises manipulating a first pair of droplets to form the first droplet interface bilayer (DIB) positioned such that the droplet of the first pair of droplets contacts the first sensing electrode and the another droplet of the first pair of droplets contacts the first bias electrode;

   and wherein forming the droplet interface bilayer between the second pair of droplets comprises manipulating a second pair of droplets to form the second droplet interface bilayer (DIB) positioned such that the droplet of the second pair of droplets contacts the second sensing electrode and the another droplet of the second pair of droplets contacts the second bias electrode.

9. The method of any preceding claim wherein the transporter species comprises a nanopore

10. A method of determining a characteristic of a first pair of droplets in an electrowetting on dielectric microfluidic device substantially free from the influences of interference, comprising;

    forming a droplet interface bilayer between a first pair of droplets;

    contacting a droplet of the first pair of droplets with a first sensing electrode and contacting another droplet of the first pair of droplets with a first bias electrode;

    measuring a first signal between the first bias electrode and the first sensing electrode for the first pair of droplets;

    inserting a nanopore into the droplet interface bilayer of the first pair of droplets;

    measuring a second signal between the first bias electrode and the first sensing electrode due to transport of a target species through the nanopore of the first pair of droplets;

    determining a difference between the second measured signal from the first pair of droplets and the first measured signal from the first pair of droplets;

and

reporting the second measured signal from the first pair of droplets and the first measured signal from the first pair of droplets as being the characteristic of the first pair of droplets, substantially free from interference.

11. The method of claim 10, wherein the first signal of the first pair of droplets comprises a first component of interference and the measured second signal of the first pair of droplets comprises a signal component of interest and a second signal component of interference.

12. The method of claim 11, wherein the signal component of interest in the measured second signal of the first pair of droplets is indicative of passage of a first species through the nanopore.

13. The method of claim 12, further comprising applying a potential across the nanopore in the droplet interface bilayer of the first pair of droplets to thereby promote passage of the first species through the nanopore.

14. The method of claim 9 to 13, wherein the nanopore is selective for the transfer of nucleic acid between one droplet and the other droplet of the first pair of droplets.

15. The method of claim 9 to 13, wherein the nanopore is selective for the transfer of protein between one droplet and the other droplet of the pair of droplets.

16. The method of claim 9 to 13, wherein the nanopore is selective for the transfer of at least one ionic species between one droplet and the other droplet of the pair of droplets.

Figure 1

Figure 2

Figure 3a

Data link 7

Electrical
Connector
8

Processor
3b

EWOD
Control
Electronics
3a

Droplet 4

Software
3c

Actuation data
Signals  5

Electrode array
38

Sensor data signals
9

2

Figure 3b

Data link 7

Processor
3b

Software
3c

Sensing
Control
electronics
3d

300

Amplifier

200

Differenti
al Current
Sensor

36

72

2

4

Sensor data signals 9

8

EWOD
control
electronics
3a

Actuation data Signals  5

Figure 4a

Reference electrode 28

Bias electrode 100

Guard electrode 130

36

Sense electrode A 110     Sense electrode B 120

Figure 4b

Reference electrode 28

36

Sense electrode A 110

Droplet pair

Sense electrode B 120

Bias electrode 100

Figure 4c

Reference electrode 28

Sense electrode A 110

Sense electrode B 120

Bias electrode 100

Connector 101

36

Droplet pair

140        150

Figure 5

Figure 6a

Droplet A

Figure 6b

Droplet A

Figure 7a

Figure 7b

Figure 7c

Figure 7d

Figure 8

Figure 9

Figure 10

Figure 11

Bias electrode 100    Bias electrode 100'

Droplet A

Sensing    Open pore

$V_{B1}$    $V_{B2}$

Droplet B

$V_{CM}$    $C_P$    $C_P$    $V_{CM}$

Sense
Electrode A 110    Sense
Electrode B
120

$I_{Sense}$    $I_{Ref}$

200    300

Differential Current Sensor    $V_{Ref}$    Amplifier    $V_{OUT}$

$V_{Sense}$

Figure 12

Sense Electrode A 120

Droplet R

Bias electrode 100

Droplet Q

30

Droplet S

Sense
Electrode B 110

$V_B$

$V_{CM}$

$I_{Ref}$

$I_{Sense}$

200

Differenti
al Current
Sensor

$V_{Ref}$

$V_{Sense}$

300

Amplifier

$V_{OUT}$

Figure 13A

Droplet A    Droplet B

Figure 13B

Droplet A    Droplet B

Europäisches
Patentamt

European
Patent Office

Office européen
des brevets

# EUROPEAN SEARCH REPORT

Application Number

EP 19 20 5485

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JASON L. POULOS ET AL: "Electrowetting on dielectric-based microfluidics for integrated lipid bilayer formation and measurement", APPLIED PHYSICS LETTERS, vol. 95, no. 1, 6 July 2009 (2009-07-06), page 013706, XP055568036, US ISSN: 0003-6951, DOI: 10.1063/1.3167283 * figures 1,2 * | 1-16 | INV. B01L3/00 C12Q1/6869 G01N33/543 |
| Y | Srikoundinya Punnamaraju: "Voltage and Photo Induced Effects in Droplet-Interface-Bilayer Lipid Membranes", PhD Thesis , 8 November 2011 (2011-11-08), pages 1-160, XP055611389, Cincinnati Retrieved from the Internet: URL:https://etd.ohiolink.edu/!etd.send_file?accession=ucin1321648604&disposition=attachment [retrieved on 2019-08-07] * pages 63-65; figures 4.3, 4.4 * | 1-16 | |
| A | SRIKOUNDINYA PUNNAMARAJU ET AL: "Voltage Control of Droplet Interface Bilayer Lipid Membrane Dimensions", LANGMUIR, vol. 27, no. 2, 18 January 2011 (2011-01-18), pages 618-626, XP055672758, US ISSN: 0743-7463, DOI: 10.1021/la1036508 * page 622; figure 7a * | 1-16 | TECHNICAL FIELDS SEARCHED (IPC) B01L G01N C12Q |

-/--

The present search report has been drawn up for all claims

| Place of search | Date of completion of the search | Examiner |
|---|---|---|
| The Hague | 5 March 2020 | Campbell, Paul |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

| | Europäisches Patentamt European Patent Office Office européen des brevets | **EUROPEAN SEARCH REPORT** | Application Number EP 19 20 5485 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YI-YING LIN ET AL: "Formation of droplets interface bilayer by coplanar EWOD device", NANO/MICRO ENGINEERED AND MOLECULAR SYSTEMS (NEMS), 2011 IEEE INTERNATIONAL CONFERENCE ON, IEEE, 20 February 2011 (2011-02-20), pages 964-967, XP031966068, DOI: 10.1109/NEMS.2011.6017515 ISBN: 978-1-61284-775-7 * pages 966-967; figure 4 * ----- | 1-16 | |
| | | | TECHNICAL FIELDS SEARCHED (IPC) |

The present search report has been drawn up for all claims

| Place of search | Date of completion of the search | Examiner |
|---|---|---|
| The Hague | 5 March 2020 | Campbell, Paul |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6911132 B, Pamula
- US 6565727 B, Shenderov
- US 7163612 B, Sterling
- US 20140194305 A
- WO 2019126715 A1
- US 8173000 B
- US 10078986 B
- US 5795782 A

**Non-patent literature cited in the description**

- **R. B. FAIR.** *Microfluid Nanofluid,* 2007, vol. 3, 245-281
- **SCHETRZER.** *Sensors and Actuators,* 2010, vol. B 145, 340-347
- *Sensors and Actuators B: Chemical,* 2013, vol. 185, 530-542
- **AQUAPORIN Z.** *Proceedings of the National Academy of Sciences,* 2007, vol. 104, 20719-20724
- **JETHA N ; WIGGIN M ; MARZIALI A.** Forming an alpha-hemolysin nanopore for single-molecule analysis. *Methods Mol Biol.,* 2009, vol. 544, 113-127
- **CHEN et al.** *RSC Advances,* 2013, vol. 3, 4473